# EUROPÄISCHE PATENTSCHRIFT

(11) **EP 3 364 170 B1**
(45) Veröffentlichungstag und Bekanntmachung des Hinweises auf die Patenterteilung: **22.11.2023**
(21) Anmeldenummer: 18157812.1
(22) Anmeldetag: 21.02.2018
(51) Int. Cl.: G01N 21/39

(54) **MESSUNG DER GASKONZENTRATION IN EINEM BEHÄLTER**
MEASURING THE CONCENTRATION OF GASES IN A CONTAINER
MESURE DE LA CONCENTRATION DE GAZ DANS UN RÉCIPIENT

(30) Priorität: 21.02.2017 AT 501382017
(43) Veröffentlichungstag der Anmeldung: 22.08.2018
(73) Patentinhaber: ACM GmbH, 1140 Wien (AT)
(72) Erfinder: HARRAUER, Eduard, 1140 Wien (AT)
(74) Vertreter: SONN Patentanwälte GmbH & Co KG

(56) Entgegenhaltungen:
- EP-A1- 2 610 608
- WO-A1-87/07018
- WO-A1-2016/156622
- DE-A1- 19 840 345
- DE-A1-102013 201 459
- US-A1- 2008 092 648
- MCMANUS J B ET AL: "Compact Quantum Cascade Laser Instrument for Rapid, High Sensitivity Measurements of Trace Gases in Air", SENSORS, 2007 IEEE, IEEE, PI, 28. Oktober 2007 (2007-10-28), Seiten 1341-1344, XP031221319, ISBN: 978-1-4244-1261-7

## Beschreibung

Die Erfindung betrifft ein Verfahren zur Ermittlung der Konzentration eines vorbestimmten Gases in einer Probe in einem geschlossenen Probenbehälter, das die Merkmale von Anspruch 1 aufweist.

Außerdem betrifft die Erfindung eine Vorrichtung zur Ermittlung der Konzentration eines vorbestimmten Gases in einer Probe in einem geschlossenen Probenbehälter, die die Merkmale von Anspruch 7 aufweist.

Derartige Verfahren und Vorrichtungen ermöglichen eine zerstörungsfreie Ermittlung der Konzentration eines vorbestimmten Gases in einer Probe. Diese Eigenschaft ist insbesondere bei der Qualitätsprüfung von in geschlossenen Behältern abgefüllten Getränken von Interesse. Abhängig davon, um welches Getränk es sich handelt, kann die Konzentration eines jeweils anderen Gases ermittelt werden. Die ermittelte Konzentration erlaubt beispielsweise Rückschlüsse auf die Haltbarkeit des Getränks.

Ein vergleichbares Verfahren ist beispielsweise bereits aus der WO 2012/001633 A2 und der WO 2008/053507 A2 bekannt. Darin ist jeweils ein Verfahren zur Ermittlung des Drucks und der Konzentration von Sauerstoff im Kopfbereich einer geschlossenen Weinflasche auf Basis eines gemessenen Absorptionsspektrums beschrieben. Die Kenntnis des Sauerstoffgehalts in einer Weinflasche ermöglicht die Feststellung der Alterung des enthaltenen Weins. Die WO 2012/001633 A2 versucht die Genauigkeit der Messung durch Verwendung zweier separater Absorptionsspektren zu erhöhen. Diese Herangehensweise erfordert eine zweite Lichtquelle und einen zweiten Detektor, was die Vorrichtung insgesamt wesentlich aufwendiger und kostspieliger macht. Die WO 2008/053507 A2 schlägt vor, zur Verbesserung der Genauigkeit Störungen, welche durch den Probenbehälter (d.h. die Weinflasche) verursacht werden, mithilfe vorab ermittelter Referenzparameter des Behälters zu kompensieren. Die WO 2016/156622 A1 beschreibt ein Verfahren zur Bestimmung der Integrität von pharmazeutischen Flaschen, die mit Stickstoff gefüllt werden, um eine Leckage zu ermitteln. Die Messung erfolgt auf optischem Wege mit Hilfe einer stimmbaren Laserquelle.

In der WO 2016/051341 A1 ist ein weiteres Verfahren dieser Gattung beschrieben, welches für die Untersuchung von stillen Getränken mit einem Stickstoffzuschlag verwendet werden kann (die Existenz des Stickstoffs wird allerdings als Störfaktor bezeichnet). Das Verfahren ist dabei für die Untersuchung von bewegten Flaschen angepasst und versucht die durch die Bewegung entstehenden Ungenauigkeiten mittels Auswahl geeigneter Zeitfenster für die Messungen zu vermeiden.

Schließlich zeigt die US 5 473 161 A ein entsprechendes Verfahren zur Ermittlung der CO2-Konzentration bzw. des CO2-Drucks. Sie befasst sich dabei mit der Auswahl einer geeigneten Wellenlänge für die Messung von CO2.

Die obigen Dokumente befassen sich abgesehen von den Grundlagen des gattungsbildenden Messverfahrens - wenn überhaupt - nur damit, Ungenauigkeiten durch Anpassung der Auswertung zu reduzieren, z.B. durch Zusammenführen zweier Messungen oder einer Messung mit einer Referenzmessung oder durch Selektion bevorzugter Messungen.

Es sind auch schon Verfahren bekannt, welche die Genauigkeit der Messung durch Anpassung des Messverfahrens verbessern sollen. Beispielsweise offenbart die US 9,310,295 B2 ein Verfahren, bei dem der zeitliche Verlauf des Pumpstrom, mit dem die Laserdiode angesteuert wird, hochfrequente Schwingungen aufweist, deren zentraler Wert abschnittsweise in Form einer Rampe ansteigen. Die Gaskonzentration kann dann aus der Amplitude einer bestimmten Frequenzkomponente des vom Lichtsensor gemessenen Signals ermittelt werden, wodurch wellenlängenunabhängige Störungen der gemessenen Gesamtintensität ausgeblendet werden können und so die Genauigkeit der Messung verbessert wird. Unmittelbar vor Beginn des im Mittel rampenartigen Verlaufs kann der Pumpstrom um einen konstanten zentralen Wert (nachfolgend kurz als "Offset" bezeichnet) schwingen. Dieser Offset kann so gewählt sein, dass der Pumpstrom in dem betreffenden Abschnitt bei oder oberhalb eines Schwellwerts der Laserdiode bleibt. Aufgrund der Schwingungen des Pumpstroms wird der Offset so weit unterhalb des zentralen Werts am Beginn der Rampe gewählt, dass der Pumpstrom in diesem Abschnitt nur im Moment eines Maximums diesen zentralen Wert am Beginn der Rampe erreicht (d.h. im Wesentlichen eine Schwingungsamplitude darunter). D.h. der Pumpstrom ist in dem Abschnitt vor Beginn der Rampe nicht konstant und außerdem praktisch immer unterhalb des zentralen Werts am Beginn der Rampe. Aufgrund der bei diesem Verfahren bewusst eingeführten Schwankungen des Pumpstroms wird die Laserdiode und mit ihr unweigerlich auch die Temperaturregelung des Dioden-Lasers belastet, was deren Lebensdauer reduziert. Außerdem wirken sich diese Schwankungen negativ auf die Reproduzierbarkeit des Verlaufs im Abschnitt der Rampe aus, weil die von der Laserdiode erzeugte Wärme aufgrund des Sprungs des zentralen Werts zwischen dem konstanten Offset und dem Beginn der Rampe ebenfalls sprunghaft ansteigt, was sich abhängig von unterschiedlichen Umgebungsbedingungen (und gegebenenfalls abhängig von der Übertragungsfunktion einer Temperaturregelung) unterschiedlich auf die Temperatur und somit auch die Wellenlänge des emittierten Laserlichts auswirkt. Abhängig von der Dauer der Rampe können solche Auswirkungen den gesamten Rampenverlauf betreffen.

Ein Pumpstrom mit einer Rampe ohne überlagerte hochfrequente Signalkomponenten ist beispielsweise in WO 2013/036378 A1 gezeigt. Allerdings wird in dem der Rampe vorangehenden Abschnitt kein Pumpstrom an die Laserdiode angelegt, sodass ein Sprung des Stromwerts vorgesehen ist, welcher die oben erläuterten Auswirkungen auf die Reproduzierbarkeit der Wellenlänge des emittierten Laserlichts hat.

Schließlich befasst sich auch die US 2008/0123713 A1 mit der Ansteuerung des Dioden-Lasers. Dabei ist ein einer Stromrampe vorangehender initialer Abschnitt vorgesehen, in dem ein - nicht näher angegebener - Pumpstrom deutlich unterhalb des Schwellenstroms der Laserdiode an die Laserdiode angelegt wird. Zu Beginn der Rampe wird der Strom rasch zum oder über den Schwellenstrom hochgefahren, womit die oben erläuterten Nachteile auch hier zum tragen kommen.

Darüber hinaus sind Verfahren bekannt, die tatsächlich weniger eine präzise Messung einer Konzentration als den Nachweis eines vorbestimmten Gases zur Aufgabe haben: Die WO 87/070181 betrifft ein Verfahren zur Messung der Konzentration von Sauerstoff in einer Probe. Im Einzelnen wird die direkte Messung einer relativen Amplitude einer Absorptionslinie von Sauerstoff beschrieben. Wie daraus die Konzentration von Sauerstoff präzise ermittelt werden kann, ist nicht genauer offenbart. Insbesondere kann die Anwesenheit von Wasserdampf in der Probe die nur anhand der Absorptionslinie ermittelte Konzentration verfälschen.

Darüber hinaus sind Messungen von Proben in einer Prozessgasleitung und somit nicht in einem geschlossenen Behälter bekannt: Die DE 10 2013 213 458 A1 zeigt ein Verfahren zur Messung der Konzentration einer infrarotaktiven Gaskomponente in einem eine Prozessgasleitung durchströmenden Messgas. Die DE 10 2000 11079 342 B3 betrifft eine Anwendung zur Überwachung eines Prozessgases in einer Prozessgasleitung mit einem Laser-Spektrometer für die Verwendung bei der optischen Gasanalyse.

Die US 2008/092648 A1 beschreibt ein Verfahren und einen Apparat zum Messen der Wasserdampfkonzentration von Kühlmitteln mithilfe eines Dioden-Lasers und eines Detektors, wobei das zu untersuchende Kühlmittel sich in einer Probenzelle befindet, dessen Temperatur gemessen und zur Berechnung der Wasserdampfkonzentration verwendet wird. Hierbei wird die Wellenlänge des vom Dioden-Laser emittierten Laserstrahls durch Änderung des Pumpstroms bei konstant gehaltener Temperatur zeitlich variiert und die Intensität des aus der Probezelle austretenden Laserstrahls zur Berechnung der Wasserdampfkonzentration ermittelt.

Die DE 10 2013 201459 A1 beschreibt ein Verfahren zur Messung der Konzentration einer Gaskomponente in einem Messgas, mithilfe einer Laserdiode und eines Detektors, wobei die Laserdiode periodisch entsprechend einer vorgegebenen rampenförmigen Strom-Zeit-Funktion angesteuert wird, um die Absorptionslinie der Gaskomponente wellenlängenabhängig abzutasten. Ein zweiter Signalgenerator erzeugt ein sinusförmiges Signal, mit dem in einem Summierglied die rampenförmige Strom-Zeit-Funktion moduliert wird. Nach der Strom-Zeit-Funktion wird die Laserdiode mit einem Burstsignal angesteuert, um die an der Stelle der Absorptionslinie detektierte Lichtintensität des Lichts mit der an der Stelle des Burstsignals detektierte Intensität zu normalisieren.

Die DE 198 40 345 A1 offenbart ein Verfahren und eine Vorrichtung zum quantitativen Aufspüren eines vorgegebenen Gases in einer Gasprobe. Dabei wird der Lasersteuerstrom eines stimmbaren Diodenlasers variiert, indem einem periodisch durchgestimmten Gleichstromanteil ein sinusförmiger Modulationsstrom überlagert wird und die Gasprobe mit dem Laserstrahl des Diodenlasers durchfahren. Die Konzentration des vorgegebenen Gases wird mithilfe des Gleichstromanteils des Lasersteuerstroms gemessenen Minimum und Maximum der zweiten Harmonischen bestimmt, wobei die Temperatur in die Berechnung der zweiten Harmonischen einfließt.

Es ist eine Aufgabe der Erfindung, die Konzentration einer Gaskomponente ohne Absorptionslinien möglichst genau zu messen.

Das erfindungsgemäße Verfahren der eingangs angeführten Art sieht vor, dass das vorbestimmte Gas Stickstoff ist, wobei eine Linienverbreiterung in dem aus der Intensitätsverteilung in Abhängigkeit vom Pumpstrom ermittelten Absorptionsspektrum ermittelt und von dieser Linienverbreiterung auf den Partialdruck von Wasserdampf in der Probe geschlossen wird, wobei die Berechnung der Stickstoffkonzentration einen kalibrierten Parameter umfasst, dessen Wert zuvor durch Kalibrierung mit zumindest einer Referenzprobe mit einer bekannten Stickstoffkonzentration ermittelt wurde .

Die erfahrungsgemäße Vorrichtung der eingangs angeführten Art sieht vor, dass sie zur Ermittlung der Stickstoffkonzentration in einer Probe in einer geschlossenen Flasche geeignet ist, und dass die Auswertungseinheit eingerichtet ist, eine Linienverbreiterung in einem aus der gemessenen Intensität in Abhängigkeit vom Pumpstrom ermittelten Absorptionsspektrum zu ermitteln und von dieser Linienverbreiterung auf den Partialdruck von Wasserdampf in der Probe zu schließen, wobei die Berechnung der Stickstoffkonzentration einen kalibrierten Parameter umfasst, dessen Wert durch Kalibrierung mit zumindest einer Referenzprobe mit einer bekannten Stickstoffkonzentration ermittelt wurde.

Die Ermittlung der Konzentration eines vorbestimmten Gases in einer Probe erfolgt dabei durch Berechnung auf Basis von Messungen physikalischer Größen. Im Einzelnen wird ein elektronisches Signal des Lichtsensors, auf den das durch die Probe transmittierte Laserlicht auftrifft, verarbeitet. Dieses Signal repräsentiert die Intensität des auf den Lichtsensor auftreffenden Lichts. Aus der Konzentration des Gases kann bei bekanntem Gesamtdruck der Partialdruck des Gases in der Probe geschätzt werden.

Das vorbestimmte Gas ist Stickstoff. Die Probe ist vorzugsweise ein Volumen oder Teilvolumen in einem Behälter, welches ein Gasgemisch und Flüssigkeiten in Form von Dampf enthält. Die Ermittlung der Stickstoffkonzentration basiert dabei auf der Erkenntnis, dass der messbare Partialdruck von Wasserdampf in der Probe auch proportional zum Partialdruck und somit der Konzentration von Stickstoff in der Probe ist. Das Verhältnis der Partialdrücke kann durch einen Gewichtungsfaktor abgeschätzt werden, welcher vom Verhältnis der Molekülmassen abhängt.

Die Temperaturmessung kann insbesondere eine pyrometrische Messung (z.B. mit einem Infrarot-Temperaturmessgerät) sein. Die gemessene Temperatur kann zur Kompensation von Abweichungen des gemessenen Absorptionsspektrums von einem unter Standardbedingungen (STP) gemessenen Absorptionsspektrum verwendet werden. Insbesondere kann diese Temperatur direkt am Messgut zur Kompensationsberechnung des ermittelten Innendruckes (z.B. von Stickstoff) auf 20°C (P20) benützt werden. Beispielsweise hängt der Anteil von Wasserdampf in der Probe auch von der Temperatur der Probe ab, die aus der gemessenen Temperatur geschätzt werden kann. Auf diese Weise hat die Temperatur der Probe einen Einfluss auf das gemessenen Absorptionsspektrum, welcher auf Grundlage der gemessenen Temperatur kompensiert werden kann, um die Genauigkeit der Konzentrationsmessung zu erhöhen.

Der von dem vorliegenden Verfahren verwendete Zusammenhang zwischen dem zeitlichen Verlauf des Pumpstroms und dem zeitlichen Verlauf der gemessenen Intensität entspricht im Wesentlichen einem Absorptionsspektrum der Probe. Verfahren zur Ermittlung solcher Absorptionsspektren sind unter der Kurzbezeichnung TDLAS (Tunable Diode Laser Absorption Spectroscopy) bekannt. Das Absorptionsspektrum kann dabei aus der durch die Probe transmittierten und gemessenen relativen Intensität als Funktion der Wellenlänge des emittierten Lichts ermittelt werden. Die Konzentration von Stickstoff wird ausgehend von dem Absorptionsspektrum und unter Anwendung des Gesetzes von Lambert-Beer ermittelt, d.h. auf Basis der Verbreiterung einer Absorptionslinie.

Als Laserdiode kann insbesondere eine DFB (distributed feedback) Diode vorgesehen sein. Bei einem stimmbaren Dioden-Laser ist die Wellenlänge des emittierten Lichts einstellbar, insbesondere durch Veränderung des Stroms und/oder der Umgebungstemperatur. Die Umgebungstemperatur kann beispielsweise mit einem thermisch mit der Laserdiode verbundenen Peltier-Element. Die Wellenlänge des emittierten Lichts wird insbesondere durch die zentrale Wellenlänge des von der Laserdiode emittierten Spektrums charakterisiert.

Der Pumpstrom ist der Strom durch die Laserdiode. Als Grundstrom wird bei dem vorliegenden Verfahren ein Strom bezeichnet, dessen Betrag größer Null ist. Vorzugsweise sind der Grundstrom und das Minimum des Betrags des Pumpstroms im Betrieb gleich; d.h. der Betrag des Pumpstroms sinkt im Betrieb nicht unter den Grundstrom. Als Stromrampe oder kurz Rampe wird ein Strom bezeichnet, der ausgehend vom einem Anfangsstrom kontinuierlich und/oder monoton, vorzugsweise streng monoton, hin zu einem Grenzstrom, welcher im Betrag größer als der Anfangsstrom ist, zeitlich geändert wird. Kontinuierlich bedeutet in diesem Zusammenhang, dass der zeitliche Verlauf kein Sprünge, die im Vergleich zu der Differenz zwischen Grenzstrom und Grundstrom signifikant sind (z.B. größer als 10 %), aufweist. Bei dem vorliegenden Verfahren ist der Anfangsstrom der Rampe gleich dem Grundstrom, d.h. die Rampe beginnt ausgehend vom Grundstrom. Vorzugsweise entspricht der Grundstrom einem unteren Stromgrenzwert (d.h. dem niedrigsten Strom im Verlauf des Pumpstroms) und der Grenzstrom einem oberen Stromgrenzwert (d.h. dem höchsten Strom im Verlauf des Pumpstroms). Der Grenzstrom kann abhängig vom verwendeten Wellenlängenbereich, der entsprechend verwendeten Laserdiode und der Qualität dieser Laserdiode zwischen 70 und 150 mA (Milliampere), vorzugsweise zwischen 80 und 110 mA, betragen.

Die zeitliche Dauer der Rampe und somit des zweiten zeitlichen Abschnitts kann vorab festgelegt sein, wobei beispielsweise ein Wert zwischen 1 und 100 ms (Millisekunden), vorzugsweise zwischen 5 und 50 ms, insbesondere von 15 ms, festgelegt werden kann. Die zeitliche Dauer des ersten zeitlichen Abschnitts ist vorzugsweise länger als die zeitliche Dauer des zweiten zeitlichen Abschnitts kann vorab festgelegt sein, wobei beispielsweise ein Wert festgelegt werden kann, der zumindest doppelt so lang, insbesondere zumindest viermal so lang, relativ zur zeitlichen Dauer des zweiten zeitlichen Abschnitts ist. Die zeitliche Dauer des ersten zeitlichen Abschnitts kann beispielsweise zwischen 2 und 500 ms, vorzugsweise zwischen 25 und 250 ms, insbesondere zwischen 80 und 90 ms betragen.

Der erste und zweite zeitliche Abschnitt des Pumpstroms können periodisch wiederholt werden, sodass der Pumpstrom periodisch gepulst ist. Eine Periode des Pumpstroms umfasst dabei zumindest einen ersten zeitlichen Abschnitt und einen unmittelbar auf den ersten zeitlichen Abschnitt folgenden zweiten zeitlichen Abschnitt. Es kann ein dritter zeitlicher Abschnitt nach dem zweiten zeitlichen Abschnitt vorgesehen sein, wobei der Pumpstrom im dritten zeitlichen Abschnitt zum Grundstrom hin abnimmt. Die zeitliche Dauer einer Periode des Pumpstroms kann beispielsweise zwischen 3 und 600 ms, vorzugsweise zwischen 30 und 300 ms, insbesondere von 100 ms, betragen.

Weil die Stromrampe des Pumpstroms der Laserdiode im zweiten zeitlichen Abschnitt des vorliegenden Verfahrens von dem Grundstrom ausgeht, wird die Temperaturänderung des Dioden-Lasers zu Beginn der Rampe minimiert. Dadurch kann ein im Vergleich zum Stand der Technik geringerer Stress bei der Ansteuerung des Dioden-Lasers, insbesondere im Zusammenhang mit der Temperaturregelung (z.B. Regelung eines Peltier-Elements), erzielt werden. Der geringere Stress kann die Langlebigkeit der Vorrichtung verbessern. Für die Temperaturregelung bedeutet eine relativ geringe Temperaturänderung des Dioden-Lasers zu Beginn der Rampe, dass die Regelabweichung gering bleibt und somit im Allgemeinen auch der Regelfehler reduziert wird. Das verbessert die Reproduzierbarkeit der Temperatur des Dioden und folglich der emittierten Wellenlänge und somit letztlich auch die Reproduzierbarkeit der durchgeführten Messungen, insbesondere bei schwankender Umgebungstemperatur.

Darüber hinaus hat es sich als vorteilhaft herausgestellt, dass der Grundstrom größer oder gleich dem Laserschwellwert der Laserdiode ist. Dementsprechend kann die Steuerungseinheit eingerichtet sein, als Grundstrom einen Pumpstrom größer oder gleich dem Laserschwellwert der Laserdiode vorzugeben. Vorzugsweise entspricht der Grundstrom im Wesentlichen dem Laserschwellwert der Laserdiode. In diesem Fall emittiert die Laserdiode bereits im ersten zeitlichen Abschnitt Laserlicht und befindet sich daher im selben Betriebsmodus wie während der Messung, d.h. sie "lasert". Ein Durchgang des Pumpstroms durch den Laserschwellwert und den resultierenden Wechsel des Betriebsmodus der Laserdiode (nicht-lasern/lasern) kann eine sprunghafte Änderung der Temperatur im Laser verursachen. Eine solche Änderung der Temperatur unmittelbar bevor die Emission von für die Messung relevanten Photonen (d.h. bei für die Messung relevanten Wellenlängen) im zweiten Abschnitt beginnt, wäre nachteilig und kann durch die oben genannte Maßnahme vermieden werden.

Gemäß einer bevorzugten Ausführungsform kann der Grundstrom größer oder gleich 1 mA, vorzugsweise zwischen 2 und 10 mA, insbesondere zwischen 3 und 5 mA, betragen. Dementsprechend kann die Steuerungseinheit eingerichtet sein, als Grundstrom einen Pumpstrom größer oder gleich 1 mA, vorzugsweise zwischen 2 und 10 mA, insbesondere zwischen 3 und 5 mA, vorzugeben.

Der Pumpstrom kann nach dem zweiten zeitlichen Abschnitt einen dritten zeitlichen Abschnitt aufweisen, wobei der Pumpstrom im dritten zeitlichen Abschnitt in zumindest zwei Stufen zum Grundstrom hin abnimmt. Dementsprechend kann die Steuerungseinheit eingerichtet sein, während eines dritten zeitlichen Abschnitts nach dem zweiten zeitlichen Abschnitt einen in zumindest zwei Stufen zum Grundstrom hin abnehmenden Pumpstrom vorzugeben. Als Stufe wird in diesem Zusammenhang eine Änderung des Pumpstroms bezeichnet, welche abwechselnd mit verschiedenen Geschwindigkeiten erfolgt. Insbesondere kann der Pumpstrom zwischen vergleichsweise steilen Flanken der einzelnen Stufen für jeweils mindestens 5 µs (Mikrosekunden), vorzugsweise mindestens 15 ps, insbesondere mindestens 30 ps, im Wesentlichen konstant gehalten werden. Durch diese stufenweise Änderung wird die Laserdiode einem geringeren momentanen Stress ausgesetzt als bei einem einzigen Sprung auf den Grundstrom. Insbesondere kann durch diese Art der Steuerung die mittlere Betriebstemperatur der Laserdiode vergleichsweise stabil gehalten werden, wodurch die Laserintensität geringeren Schwankungen als bei einem einzigen Sprung oder einer entsprechend steilen Flanke ausgesetzt ist. Steile Flanken führen immer auch zum Temperaturstress und somit zu einer erhöhten Anforderung an eine Temperaturregelung (z.B. eine Peltierregelung). Unter diesen Umständen führt eine schwankende Umgebungstemperatur zu einer Verschlechterung der Messergebnisse. Mit der vorgeschlagenen Steuerung kann der Einfluss von Schwankungen der Umgebungstemperatur minimiert werden. Alternativ kann der Strom im Anschluss an die Stromrampe im zweiten Abschnitt in einem Sprung auf den Grundstrom geregelt werden.

Außerdem ist es vorteilhaft, wenn das von dem Dioden-Laser emittierte Licht vor einem Eintritt in die Probe und/oder nach einem Austritt aus der Probe gestreut wird. Dementsprechend kann die vorliegende Vorrichtung zumindest ein Diffusionselement umfassen, welches zur Streuung des von der Laserdiode abgestrahlten Lichts vor dem Eintritt in die Probe und/oder nach dem Austritt aus der Probe eingerichtet ist. Die Streuung kann insbesondere mit einem oder mehreren Diffusionselementen erzielt werden, durch die das Licht transmittiert oder an denen es reflektiert wird. Das Ausmaß der Streuung bzw. das Diffusionselement (z.B. dessen Zusammensetzung oder Struktur) sind eingerichtet, lokale Unterschiede in der Intensität des gestreuten Lichts auszugleichen. Es hat sich herausgestellt, dass das von einer Laserdiode abgestrahlte Licht eine vom Raumwinkel abhängige inhomogene Verteilung der Intensität aufweist. Diese Verteilung ändert sich zusätzlich in Abhängigkeit von der emittierten Wellenlänge. Durch die vorgeschlagene Streuung kann diese Verteilung zumindest teilweise gemischt und somit an eine gleichmäßigere Verteilung, insbesondere eine Normalverteilung, angenähert werden, die für alle Wellenlängen ungefähr gleich ist. Das Diffusionselement kann eine im Wesentlichen homogene Diffusion der transmittierten Strahlung bewirken (z.B. eine Mattscheibe oder ein Butterbrotpapier) und/oder lokal unterschiedliche Diffusions- und Absorptionseigenschaften aufweisen (etwa in Form einer Blende, welche durch eine lokale Färbung eines ansonsten transparenten Elements erzielt wird, z.B. Glas mit Farbtupfer). Das Ausmaß der Streuung kann beispielsweise durch Wahl eines geeigneten Gratings festgelegt werden, wobei der Fachmann die Homogenität gegen die verbleibende Intensität der transmittierten Strahlung abwägen wird. Durch die höhere Homogenität des Lichts wird das Verfahren robuster (d.h. unempfindlicher) gegenüber Wechselwirkungen von inhomogenen optischen Eigenschaften des Probenbehälters (welche z.B. durch lokale Variationen des Behältermaterials oder der Behältergeometrie, insbesondere der Wandstärke, verursacht werden können) mit je nach Wellenlänge unterschiedlichen Intensitätsverteilungen des emittierten Laserlichts. Ein Effekt der Streuung ist, dass lokale Spitzen (Maxima) der Intensität des Laserlichts ausgeglichen werden können. Ohne einen solchen Ausgleich können solche Spitzen zu einer Empfindlichkeit der Messung für lokale Variationen der optischen Eigenschaften des Probenbehälters führen. Besonders vorteilhaft ist die Streuung bei einer von der Wellenlänge abhängigen Inhomogenität des von der Laserdiode abgestrahlten Lichts, d.h. wenn die Spitzen der Intensitätsverteilung bei unterschiedlichen Wellenlängen bei unterschiedlichen Positionen oder Raumwinkeln auftreten.

In diesem Zusammenhang ist es günstig, wenn das von dem Dioden-Laser emittierte Licht fokussiert wird bevor oder unmittelbar nachdem es gestreut wird. Dementsprechend kann die Vorrichtung zumindest eine Sammellinse umfassen, welche zur Fokussierung des von der Laserdiode abgestrahlten Lichts vor der Streuung an oder in dem Diffusionselement oder, insbesondere unmittelbar, danach eingerichtet ist. Die Sammellinse kann insbesondere zusätzlich zu der Optik des Dioden-Lasers, welche in der Regel auf einen parallelen Strahlengang des abgestrahlten Lichts abgestimmt ist, zur Brechung des Laserlichts vorgesehen sein. Die Sammellinse ist dabei dem Diffusionselement im Strahlengang vorzugsweise vorgeordnet, d.h. zwischen Diffusionselement und Dioden-Laser angeordnet. Eine Fokussierung des Lichts, insbesondere bevor es auf das Diffusionselement trifft, wirkt einem durch die Streuung verursachten Intensitätsverlust entgegen und erlaubt daher eine stärkere Streuung und somit eine bessere Mischung und Homogenität (d.h. noch geringere lokale Schwankungen der Intensitätsverteilung). Ein Intensitätsverlust wäre nachteilig, weil er das Signal der Messung und somit das Signal-RauschVerhältnis verringern und die Ungenauigkeit der Messung steigern würde.

Gemäß einer bevorzugen Ausführungsform weist das vom Dioden-Laser emittierte Laserlicht eine Wellenlänge zwischen 1300 und 2000 nm (Nanometer), vorzugsweise zwischen 1750 und 1950 nm, insbesondere zwischen 1810 und 1850 nm, auf. Dementsprechend kann der Dioden-Laser zur Emission von Laserlicht einer Wellenlänge zwischen 1300 und 2000 nm, vorzugsweise zwischen 1750 und 1950 nm, insbesondere zwischen 1810 und 1850 nm, eingerichtet sein. Für das gegenständliche Verfahren hat sich herausgestellt, dass in dem oben als bevorzugt angegebenen Wellenlängenbereich die höchste Genauigkeit der ermittelten Konzentration erreicht werden kann. Alternativ kann auch Laserlicht einer Wellenlänge zwischen 1350 und 1550 nm bzw. ein entsprechend eingerichteter Dioden-Laser verwendet werden. Die Breite des gemessenen Wellenlängenbereichs entspricht vorzugsweise mindestens der Linienbreite einer Wasserdampflinie im genannten Wellenlängenbereich.

Bei dem vorliegenden Verfahren ist das vorbestimmte Gas Stickstoff und die Berechnung der Stickstoffkonzentration umfasst einen kalibrierten Parameter, dessen Wert zuvor durch Kalibrierung mit zumindest einer Probe mit einer bekannten Stickstoffkonzentration (Referenzprobe) ermittelt wurde. Zur Kalibrierung können beispielsweise Referenzproben mit einem Partialdruck von N2 von 0,5 bis 1 bar verwendet werden. Dementsprechend ist eine bevorzugte Anwendung der vorliegenden Vorrichtung die Ermittlung der Stickstoffkonzentration in einer geschlossenen Flasche, insbesondere in einer PET-Flasche. Bei "stillen" Getränken (ohne CO2) ist es üblich, die Flaschen vor dem Verschließen zusätzlich zum Getränk mit einem Tropfen Flüssigstickstoff zu befüllen. Dadurch wird das Getränk in der PET-Flasche für mindestens sechs Monate bakteriologisch geschützt. Außerdem wird der Stapeldruck der befüllten Flaschen erhöht. Eine möglichst genaue Ermittlung der Stickstoffkonzentration erlaubt eine zuverlässige Feststellung der Haltbarkeit. Die vorgeschlagene Vorrichtung ermöglicht eine besonders genaue Ermittlung ohne dass dafür ein Öffnen der Flasche erforderlich wäre. Dadurch kann dieselbe Probe mehrmals über einen längeren Zeitraum hinweg zur Prüfung der Haltbarkeit verwendet werden und es erübrigt sich die Aufbewahrung einer Sammlung von Proben, die jeweils zu unterschiedlichen Zeitpunkten geöffnet und damit zerstört werden müssten, um die Haltbarkeit über einen längeren Zeitraum hinweg zu überwachen.

Gemäß einer besonders bevorzugten Ausführungsform der Erfindung kann bei einem Verfahren zur Ermittlung der Konzentration eines Gases in einer Probe in einem geschlossenen Behälter durch Messen eines Absorptionsspektrums der Probe mithilfe eines stimmbaren Dioden-Lasers, welcher eine Laserdiode und ein mit der Laserdiode thermisch verbundenes Peltier-Element aufweist, das Messen des Absorptionsspektrums zumindest die folgenden Schritte umfassen:
Bereitstellen eines periodisch gepulsten Pumpstroms durch die Laserdiode zur Variation der Wellenlänge des von der Laserdiode emittierten Laserlichts, wobei eine Periode des Pumpstroms zumindest einen ersten Abschnitt und einen unmittelbar auf den ersten Abschnitt folgenden zweiten Abschnitt umfasst, wobei der Pumpstrom im ersten Abschnitt konstant ist und einem Grundstrom entspricht, wobei der Pumpstrom im zweiten Abschnitt kontinuierlich ansteigend zwischen einem unteren Stromgrenzwert und einem oberen Stromgrenzwert geändert wird (insbesondere in Form einer ansteigenden Rampe),
Detektieren der Intensität des durch die Probe transmittieren Laserlichts und
Erfassen der detektierten Intensität in Abhängigkeit von einem Momentanwert des Pumpstroms,
wobei der Wert (oder Pegel) des Grundstroms dem unteren Stromgrenzwert entspricht.

Die Erfindung wird nachfolgend anhand von besonders bevorzugten Ausführungsbeispielen, auf die sie jedoch nicht beschränkt sein soll, und unter Bezugnahme auf die Zeichnungen noch weiter erläutert. Dabei zeigen im Einzelnen:
Fig. 1 schematisch eine erfindungsgemäße Vorrichtung zur Ermittlung der Konzentration eines vorbestimmten Gases in einer Probe;
Fig. 2 schematisch das optische System der Vorrichtung gemäß Fig. 1;
Fig. 3a und 3b eine simulierte geometrische Intensitätsverteilung über die Bildebene des von einem Lichtsensor aufgefangenen Lichts bei einer Vorrichtung gemäß Fig. 2 ohne Diffusionselemente (Fig. 3a) bzw. mit Diffusionselementen (Fig. 3b);
Fig. 4 schematisch eine Periode eines Pumpstroms der Laserdiode der Vorrichtung gemäß Fig. 1.

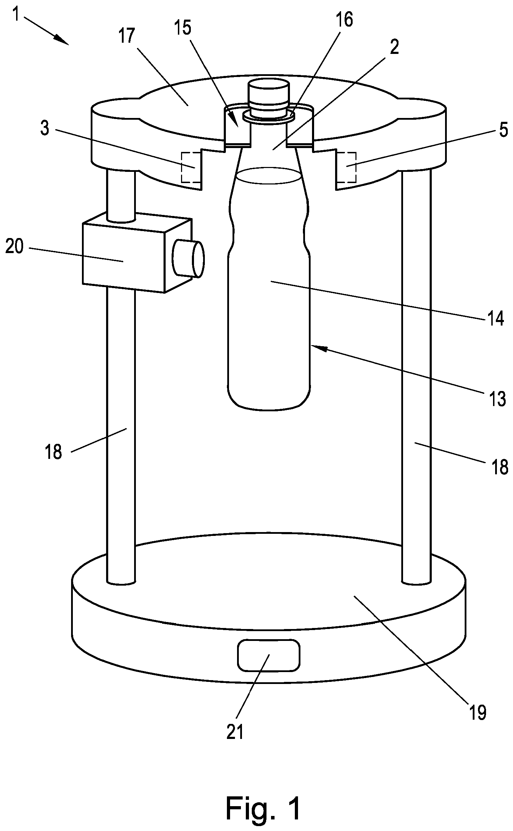

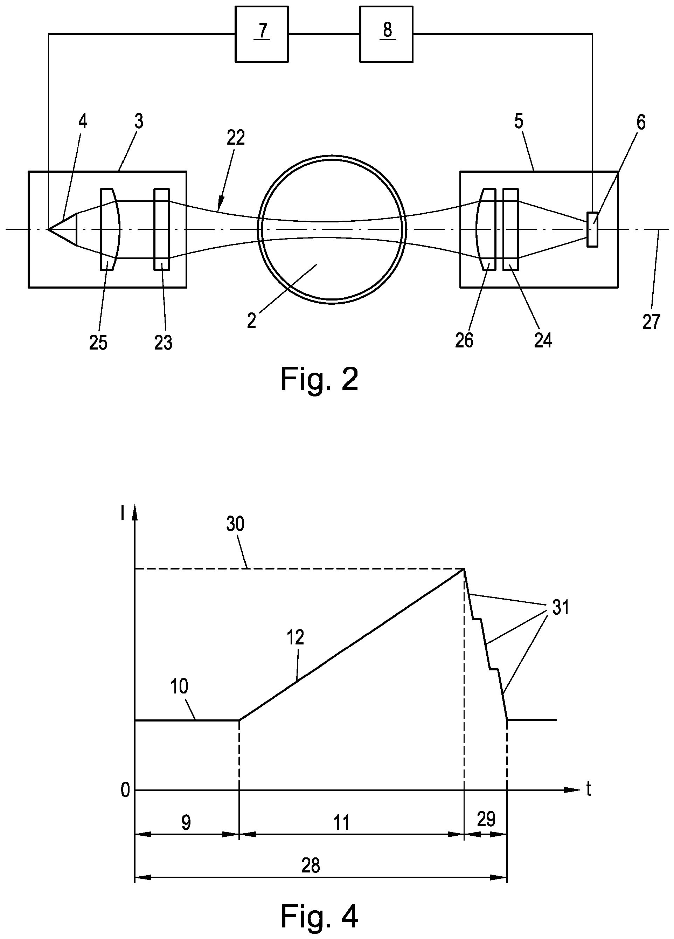

In Fig. 1 ist eine erfindungsgemäße Vorrichtung 1 zur Ermittlung der Konzentration eines vorbestimmten Gases in einer Probe 2 gezeigt. Die Vorrichtung 1 umfasst eine Lichtquelle 3 mit einem stimmbaren Dioden-Laser 4 (vgl. Fig. 2). Der Dioden-Laser 4 umfasst eine DFB (distributed feedback) Laserdiode (nicht gezeigt) zum Abstrahlen von im Wesentlichen monochromatischem Licht in die Probe 2. Der Dioden-Laser 4 ist zur Emission von Laserlicht einer Wellenlänge zwischen 1810 und 1850 nm eingerichtet, d.h. er ist zwischen 1810 und 1850 nm stimmbar.

Die Vorrichtung 1 umfasst außerdem eine Messeinheit 5 mit einem Lichtsensor 6 zur Messung der Intensität des aus der Probe 2 austretenden Lichts. Weiters umfasst die Vorrichtung 1 eine Steuerungseinheit 7 zur Steuerung des Pumpstroms der Laserdiode des Dioden-Lasers 4. Schließlich umfasst die Vorrichtung auch eine Auswertungseinheit 8. Die Steuerungseinheit 7 ist eingerichtet, den Pumpstrom der Laserdiode des Dioden-Lasers 4 zeitlich zu variieren, insbesondere periodisch zu modulieren. Im Einzelnen ist sie eingerichtet, als Pumpstrom der Laserdiode des Dioden-Lasers 4 während eines ersten zeitlichen Abschnitts 9 (vgl. Fig. 4) einen im Wesentlichen konstanten und von Null verschiedenen Grundstrom 10 vorzugeben. Dieser Grundstrom 10 entspricht im vorliegenden Beispiel dem Laserschwellwert des Dioden-Lasers 4. Die Steuerungseinheit 7 ist außerdem eingerichtet, während eines zweiten zeitlichen Abschnitts 11 eine ausgehend von dem Grundstrom 10 kontinuierlich ansteigende Stromrampe 12 als Pumpstrom der Laserdiode des Dioden-Lasers 4 vorzugeben. Weiters ist Steuerungseinheit eingerichtet, während eines dritten zeitlichen Abschnitts 29 einen in drei Stufen zum Grundstrom 10 hin abnehmenden Pumpstrom I vorzugeben. Die Auswertungseinheit 8 ist mit dem Lichtsensor 6 und mit der Steuerungseinheit 7 verbunden. Sie ist außerdem zur Berechnung der Konzentration des Gases in Abhängigkeit von dem von der Steuerungseinheit 7 vorgegebenen Pumpstrom und dem vom Lichtsensor 6 gemessenen Intensität des durch die Probe 2 transmittierten Lichts eingerichtet.

Die Probe 2 in Fig. 1 ist ein flüssigkeitsleerer Kopfraum eines Probenbehälters 13 in Form einer geschlossenen PET-Flasche, welche ein Getränk 14 (hauptsächlich Wasser) enthält. Die relative Feuchtigkeit im Kopfraum ist im Wesentlichen 100 %, d.h. es liegt hier Wasserdampf vor. Der Probenbehälter 13 ist für Licht im Infrarotbereich im Wesentlichen transparent. Er ist in einer im Wesentlichen U-förmigen Einschubhalterung 15 aufgenommen und wird von der Einschubhalterung 15 an einem Verschlusskragen 16 des Probenbehälters 13 getragen. Ein Oberteil 17 der Vorrichtung 1 ist mittels zweier Stützelemente 18 in Form von Stangen mit einem Sockel 19 verbunden. An einem der Stützelemente 18 ist unterhalb des Oberteils 17 eine Temperaturmesseinrichtung 20 in Form einer Infrarot-Temperaturmessgeräts angeordnet. Die Temperaturmesseinrichtung 20 ist zur Messung der Temperatur an einer Außenwand des Probenbehälters 13 eingerichtet. Im Betrieb der Vorrichtung 1 wird zusätzlich zum Absorptionsspektrum mit der Temperaturmesseinrichtung 20 die Temperatur an der Außenwand des Probenbehälters 13 gemessen und bei der Berechnung der Konzentration des Gases berücksichtigt. Am Sockel 19 ist ein Display 21 zur Anzeige der ermittelten Konzentration oder des ermittelten Partialdrucks vorgesehen.

In Fig. 2 ist das optische System der Vorrichtung 1 zusammen mit einem vereinfachten Strahlengang 22 sowie den nur schematisch eingezeichneten Funktionseinheiten 3, 5, 7, 8 der Vorrichtung 1 sowie deren Verbindungen untereinander dargestellt. Das optische System der Vorrichtung 1 umfasst zwei Diffusionselemente 23, 24. Die Diffusionselemente 23, 24 sind jeweils zur Transmission und Streuung des von der Laserdiode des Dioden-Lasers 4 abgestrahlten (emittierten) Lichts eingerichtet. Ein erstes Diffusionselement 23 streut das Licht vor dem Eintritt in die Probe 2. Ein zweites Diffusionselement 24 streut das Licht nach dem Austritt aus der Probe 2.

Außerdem ist in der Lichtquelle 3 und der Messeinheit 5 jeweils eine Sammellinse 25, 26 vorgesehen. Eine erste Sammellinse 25 in der Lichtquelle 3 ist zur Fokussierung des von der Laserdiode des Dioden-Lasers 4 abgestrahlten Lichts vor der Streuung am ersten Diffusionselement 23 eingerichtet. Eine zweite Sammellinse 26 in der Messeinheit 5 ist zur Fokussierung des aus der Probe 2 austretenden Lichts vor der Streuung am zweiten Diffusionselement 24 eingerichtet. Somit wird im Betrieb das von dem Dioden-Laser 4 emittierte Licht jeweils fokussiert bevor es in einem der Diffusionselemente 23, 24 gestreut wird.

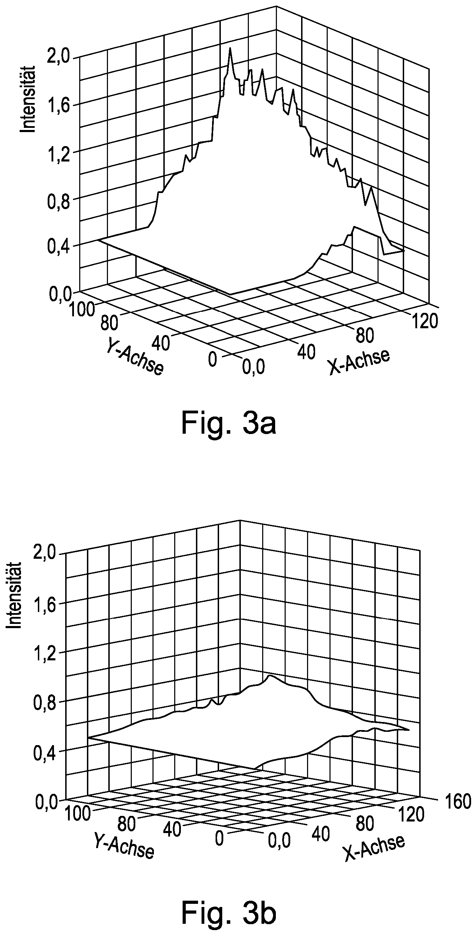

Die Wirkung des optischen Systems der Vorrichtung ist wird anhand der Fig. 3a und 3b verständlich. Fig. 3a zeigt eine an einer beispielhaften Mess- und Prüfanordnung gemessene geometrische Intensitätsverteilung der vom Dioden-Laser 4 emittierten Laserlichts in einer Bildebene normal auf die optische Achse 27 für ein optisches System, welches bis auf die Diffusionselemente 23, 24 und Sammellinsen 25, 26 dem in Fig. 2 dargestellten System entspricht. Fig. 3b zeigt im Vergleich dazu eine entsprechende geometrische Intensitätsverteilung bei Verwendung des in Fig. 2 dargestellten optischen Systems, d.h. mit Diffusionselementen 23, 24 und Sammellinsen 25, 26. Es ist eindeutig zu erkennen, dass bei der Verteilung in Fig. 3b die Intensitätsspitzen (lokalen Maxima) der Verteilung in Fig. 3a eliminiert wurden. Die Intensitätsverteilung entspricht annähernd einer Gleichverteilung in der Bildebene. Die so erzielte gleichmäßige Intensitätsverteilung bleibt bei Änderungen des Pumpstroms im Wesentlichen gleich und ist von der Bauart und Exemplar des Lasers unabhängig. Die Sammellinsen 25, 26 können außerdem zu konfiguriert sein, dass Brechungen an dem Probenbehälter zumindest teilweise kompensiert werden können.

Unter Verwendung der Vorrichtung gemäß Fig. 1 und 2 kann die Konzentration eines vorbestimmten Gases, z.B. Stickstoff, in der Probe 2 nach dem vorliegenden Verfahren bestimmt werden. Dabei wird die Wellenlänge (eigentlich die zentrale Wellenlänge des Emissionsspektrums) des vom Dioden-Laser 4 in die Probe 2 abgestrahlten, im Wesentlichen monochromatischen Lichts durch Änderung des Pumpstroms I der Laserdiode des Dioden-Lasers 4 zeitlich variiert und moduliert (vgl. Fig. 4). Anschließend wird die Intensität des aus der Probe 2 austretenden Lichts mit der Messeinheit 5 gemessen und die Konzentration des Gases in der Probe in Abhängigkeit von dem zeitlichen Verlauf des Pumpstroms I und dem zeitlichen Verlauf der gemessenen Intensität in der Auswertungseinheit 8 berechnet. Dabei wird insbesondere die Linienverbreiterung in dem aus der Intensitätsverteilung in Abhängigkeit vom Pumpstrom ermittelten Absorptionsspektrum ermittelt und von dieser Linienverbreiterung auf den Partialdruck von Wasserdampf in der Probe geschlossen. Der Zusammenhang zwischen diesem Partialdruck und dem Partialdruck des gesuchten Gases (z.B. Stickstoff) kann durch Kalibrierung vorab ermittelt und anschließend zur Messung verwendet werden. Die Kalibrierung kann beispielsweise anhand einer oder mehrerer Referenzproben mit einer bekannten Stickstoffkonzentration durchgeführt werden.

In Fig. 4 ist der zeitliche Verlauf einer Periode 28 des Pumpstroms I der Laserdiode des Dioden-Lasers 4 der Vorrichtung 1 gemäß Fig. 1 schematisch dargestellt. Die Dauer einer Periode 28 in diesem Beispiel beträgt 100 ms. Im Betrieb wird die Periode 28 in einer Schleife wiederholt, sodass sich eine Pulsfrequenz des Pumpstroms entsprechend der inversen Periodendauer ergibt, d.h. beispielsweise eine Pulsfrequenz von 10 Hz. Zur Erläuterung des zeitlichen Verlaufs des Pumpstroms I wird die Periode 28 in einen ersten zeitlichen Abschnitt 9, einen zweiten zeitlichen Abschnitt 11 und einen dritten zeitlichen Abschnitt 29 unterteilt. Die Dauer der einzelnen zeitlichen Abschnitte ist nicht proportional dargestellt.

Wie aus Fig. 4 ersichtlich entspricht der Pumpstrom I im ersten zeitlichen Abschnitt 9 einem im Wesentlichen konstanten und von Null verschiedenen Grundstrom 10. Der Grundstrom 10 entspricht ungefähr dem Laserschwellwert der Laserdiode des Dioden-Lasers 4. Somit wird die Laserdiode ständig im Laser-Betrieb angesteuert und gleichzeitig eine möglichst geringe Temperaturentwicklung in diesem Betriebsmodus erzielt. Im vorliegenden Beispiel ist der Grundstrom 5 mA. Die Dauer des ersten zeitlichen Abschnitts 9 in diesem Beispiel ist etwa 84,9 ms.

Im zweiten zeitlichen Abschnitt 11, welcher unmittelbar an den ersten zeitlichen Abschnitt 9 anschließt, wird der Pumpstrom I von der Steuerungseinheit 7 entsprechend einer ausgehend von dem Grundstrom 10 kontinuierlich ansteigenden Stromrampe 12 bis zu einem Grenzstrom 30 geändert bzw. hochgefahren. Die Dauer des zweiten zeitlichen Abschnitts 11 in diesem Beispiel ist exakt 15 ms. Im vorliegenden Beispiel ist der Grenzstrom etwa 100 mA.

Nach dem zweiten zeitlichen Abschnitt 11 folgt unmittelbar der dritte zeitliche Abschnitt 29, in dem der Pumpstrom I in drei Stufen 31 zum Grundstrom 10 hin verändert bzw. heruntergefahren wird. Die Dauer der einzelnen Stufen ist jeweils maximal 35 µs (Mikrosekunden), sodass sich insgesamt eine Dauer des dritten zeitlichen Abschnitts 29 von maximal etwa 100 µs (das sind etwa 0,1 ms) ergibt.

## Patentansprüche

1. Verfahren zur Ermittlung der Konzentration eines vorbestimmten Gases in einer Probe (2) in einem geschlossenen Probenbehälter (13) mithilfe eines stimmbaren Dioden-Lasers (4) und eines Lichtsensors (6), wobei eine Wellenlänge des vom Dioden-Laser (4) in die Probe (2) abgestrahlten, im Wesentlichen monochromatischen Lichts durch Änderung eines Pumpstroms (I) einer Laserdiode des Dioden-Lasers (4) zeitlich variiert wird, die Intensität des aus der Probe (2) austretenden Lichts gemessen wird, und die Konzentration des Gases in der Probe (2) in Abhängigkeit von dem zeitlichen Verlauf des Pumpstroms (I) und dem zeitlichen Verlauf der gemessenen Intensität berechnet wird, wobei zusätzlich zu einem Absorptionsspektrum eine Temperatur an der Außenwand des Probenbehälters (13) gemessen und bei der Berechnung der Konzentration des Gases berücksichtigt wird, und wobei der Pumpstrom (I) einen ersten zeitlichen Abschnitt (9) mit einem im Wesentlichen konstanten und von Null verschiedenen Grundstrom (10) und einen zweiten zeitlichen Abschnitt (11) mit einer ausgehend von dem Grundstrom (10) kontinuierlich ansteigenden Stromrampe (12) aufweist, wobei das vorbestimmte Gas Stickstoff ist, wobei eine Linienverbreiterung in dem aus der Intensitätsverteilung in Abhängigkeit vom Pumpstrom ermittelten Absorptionsspektrum ermittelt und von dieser Linienverbreiterung auf den Partialdruck von Wasserdampf in der Probe geschlossen wird, wobei die Berechnung der Stickstoffkonzentration einen kalibrierten Parameter umfasst, dessen Wert zuvor durch Kalibrierung mit zumindest einer Referenzprobe mit einer bekannten Stickstoffkonzentration ermittelt wurde.

2. Verfahren nach Anspruch 1, **dadurch gekennzeichnet, dass** der Grundstrom (10) größer oder gleich dem Laserschwellwert der Laserdiode ist.

3. Verfahren nach einem der Ansprüche 1 oder 2, **dadurch gekennzeichnet, dass** der Pumpstrom (I) nach dem zweiten zeitlichen Abschnitt (11) einen dritten zeitlichen Abschnitt (29) aufweist, wobei der Pumpstrom (I) im dritten zeitlichen Abschnitt (29) in zumindest zwei Stufen (31) zum Grundstrom (10) hin abnimmt.

4. Verfahren nach einem der Ansprüche 1 bis 3, **dadurch gekennzeichnet, dass** das von dem Dioden-Laser (4) emittierte Licht vor einem Eintritt in die Probe (2) und/oder nach einem Austritt aus der Probe (2) gestreut wird.

5. Verfahren nach Anspruch 4, **dadurch gekennzeichnet, dass** das von dem Dioden-Laser (4) emittierte Licht fokussiert wird bevor oder unmittelbar nachdem es gestreut wird.

6. Verfahren nach einem der Ansprüche 1 bis 5, **dadurch gekennzeichnet, dass** das vom Dioden-Laser (4) emittierte Laserlicht eine Wellenlänge zwischen 1300 und 2000 nm, vorzugsweise zwischen 1750 und 1950 nm, insbesondere zwischen 1810 und 1850 nm, aufweist.

7. Vorrichtung (1) zur Ermittlung der Stickstoffkonzentration in einer Probe (2) in einer geschlossenen Flasche, die Vorrichtung (1) umfassend einen Dioden-Laser (4) mit einer Laserdiode zum Abstrahlen von im Wesentlichen monochromatischem Licht in die Probe (2), einen Lichtsensor (6) zur Messung der Intensität des aus der Probe (2) austretenden Lichts, eine Steuerungseinheit (7) zur Steuerung des Pumpstroms (I) der Laserdiode, und eine Auswertungseinheit (8), wobei die Steuerungseinheit (7) eingerichtet ist, den Pumpstrom (I) der Laserdiode zeitlich zu variieren und wobei die Auswertungseinheit (8) mit dem Lichtsensor (6) und mit der Steuerungseinheit (7) verbunden und zur Berechnung der Stickstoffkonzentration in Abhängigkeit von dem von der Steuerungseinheit (7) vorgegebenen Pumpstrom (I) und der vom Lichtsensor (6) gemessenen Intensität eingerichtet ist, wobei die Vorrichtung (1) eine Temperaturmesseinrichtung (20) umfasst, welche zur Messung der Temperatur an einer Außenwand der Flasche eingerichtet ist, und wobei die Steuerungseinheit (7) eingerichtet ist, als Pumpstrom (I) während eines ersten zeitlichen Abschnitts (9) einen im Wesentlichen konstanten und von Null verschiedenen Grundstrom (10) vorzugeben und während eines zweiten zeitlichen Abschnitts (11) eine ausgehend von dem Grundstrom (10) kontinuierlich ansteigende Stromrampe (12) vorzugeben, wobei die Auswertungseinheit (8) eingerichtet ist, eine Linienverbreiterung in einem aus der gemessenen Intensität in Abhängigkeit vom Pumpstrom ermittelten Absorptionsspektrum zu ermitteln und von dieser Linienverbreiterung auf den Partialdruck von Wasserdampf in der Probe zu schließen, wobei die Berechnung der Stickstoffkonzentration einen kalibrierten Parameter umfasst, dessen Wert durch Kalibrierung mit zumindest einer Referenzprobe mit einer bekannten Stickstoffkonzentration ermittelt wurde.

8. Vorrichtung (1) nach Anspruch 7, **dadurch gekennzeichnet, dass** die Steuerungseinheit (7) eingerichtet ist, als Grundstrom (10) einen Pumpstrom (I) größer oder gleich einem Laserschwellwert der Laserdiode vorzugeben.

9. Vorrichtung (1) nach einem der Ansprüche 7 oder 8, **dadurch gekennzeichnet, dass** die Steuerungseinheit (7) eingerichtet ist, während eines dritten zeitlichen Abschnitts (29) nach dem zweiten zeitlichen Abschnitt (11) einen in zumindest zwei Stufen (31) zum Grundstrom (10) hin abnehmenden Pumpstrom (I) vorzugeben.

10. Vorrichtung (1) nach einem der Ansprüche 7 bis 9, **dadurch gekennzeichnet, dass** die Vorrichtung (1) zumindest ein Diffusionselement (23, 24) umfasst, welches zur Streuung des von der Laserdiode abgestrahlten Lichts vor dem Eintritt in die Probe (2) und/oder nach dem Austritt aus der Probe (2) eingerichtet ist.

11. Vorrichtung (1) nach Anspruch 10, **dadurch gekennzeichnet, dass** die Vorrichtung (1) zumindest eine Sammellinse (25, 26) umfasst, welche zur Fokussierung des von der Laserdiode abgestrahlten Lichts vor der Streuung an oder in dem Diffusionselement (23, 24) oder danach eingerichtet ist.

12. Vorrichtung (1) nach einem der Ansprüche 7 bis 11, **dadurch gekennzeichnet, dass** der Dioden-Laser (4) zur Emission von Laserlicht einer Wellenlänge zwischen 1300 und 2000 nm, vorzugsweise zwischen 1750 und 1950 nm, insbesondere zwischen 1810 und 1850 nm, eingerichtet ist.

## Claims

1. A method for determining the concentration of a predetermined gas in a sample (2) in a closed sample container (13) using a tunable diode laser (4) and a light sensor (6), wherein a wavelength of substantially monochromatic light emitted from the diode laser (4) into the sample (2) is varied in time by changing a pump current (I) of a laser diode of the diode laser (4), the intensity of the light emerging from the sample (2) is measured, and the concentration of the gas in the sample (2) is calculated as a function of the variation in time of the pump current (I) and the variation in time of the measured intensity, wherein, in addition to an absorption spectrum, a temperature at the outer wall of the sample container (13) is measured and taken into account when calculating the concentration of the gas, and wherein the pump current (I) has a first temporal portion (9) with a substantially constant and non-zero base current (10) and a second temporal portion (11) with a current ramp (12) continuously increasing from the base current (10), wherein the predetermined gas is nitrogen, wherein a line broadening is determined in the absorption spectrum determined from the intensity distribution as a function of the pump current and the partial pressure of water vapor in the sample is inferred from this line broadening, wherein the calculation of the nitrogen concentration comprises a calibrated parameter whose value has been previously determined by calibration with at least one reference sample having a known nitrogen concentration.

2. The method according to claim 1, **characterized in that** the base current (10) is greater than or equal to the laser threshold of the laser diode.

3. The method according to any of claims 1 or 2, **characterized in that** the pump current (I) has a third temporal portion (29) after the second temporal portion (11), wherein the pump current (I) decreases in the third temporal portion (29) in at least two steps (31) towards the base current (10).

4. The method according to any of claims 1 to 3, **characterized in that** the light emitted by the diode laser (4) is scattered before entering the sample (2) and/or after exiting the sample (2).

5. The method according to claim 4, **characterized in that** the light emitted by the diode laser (4) is focused before or immediately after it is scattered.

6. The method according to any of claims 1 to 5, **characterized in that** the laser light emitted by the diode laser (4) has a wavelength between 1300 and 2000 nm, preferably between 1750 and 1950 nm, particularly between 1810 and 1850 nm.

7. A device (1) for determining the nitrogen concentration in a sample (2) in a closed bottle, the device (1) comprising a diode laser (4) with a laser diode for emitting substantially monochromatic light into the sample (2), a light sensor (6) for measuring the intensity of the light emerging from the sample (2), a controller (7) for controlling the pump current (I) of the laser diode, and an evaluation unit (8), wherein the control unit (7) is configured to vary the pump current (I) of the laser diode over time, and wherein the evaluation unit (8) is connected to the light sensor (6) and to the control unit (7) and is configured to calculate the nitrogen concentration as a function of the pump current (I) predetermined by the control unit (7) and the intensity measured by the light sensor (6), wherein the device (1) comprises a temperature measuring apparatus (20) which is configured to measure the temperature at an outer wall of the bottle, and wherein the control unit (7) is configured to preset as pump current (I) during a first time portion (9) a substantially constant and non-zero basic current (10) and during a second time portion (11) a current ramp (12) which continuously increases starting from the basic current (10), wherein the evaluation unit (8) is configured to determine a line broadening in an absorption spectrum determined from the measured intensity as a function of the pump current and to infer the partial pressure of water vapor in the sample from this line broadening, wherein the calculation of nitrogen concentration comprises a calibrated parameter whose value has been determined by calibration with at least one reference sample having a known nitrogen concentration.

8. The device (1) according to claim 7, **characterized in that** the control unit (7) is configured to preset as the base current (10) a pump current (I) greater than or equal to a laser threshold value of the laser diode.

9. The device (1) according to any of claims 7 or 8, **characterized in that** the control unit (7) is configured to preset a pump current (I) decreasing in at least two stages (31) towards the base current (10) during a third temporal portion (29) after the second temporal portion (11).

10. The device (1) according to any of claims 7 to 9, **characterized in that** the device (1) comprises at least one diffusion element (23, 24) configured to scatter the light emitted by the laser diode before entering the sample (2) and/or after exiting the sample (2).

11. The device (1) according to claim 10, **characterized in that** the device (1) comprises at least one converging lens (25, 26) configured to focus the light emitted by the laser diode before scattering by or in the diffusion element (23, 24) or thereafter.

12. The device (1) according to any of claims 7 to 11, **characterized in that** the diode laser (4) is configured to emit laser light of a wavelength between 1300 and 2000 nm, preferably between 1750 and 1950 nm, particularly between 1810 and 1850 nm.

## Revendications

1. Procédé pour la détermination d'un gaz prédéfini dans un échantillon (2) dans un récipient d'échantillon (13) fermé à l'aide d'un laser à diodes (4) accordable et d'un détecteur de lumière (6), une longueur d'onde de la lumière essentiellement monochromatique, rayonnant à partir du laser à diodes (4) dans l'échantillon (2) variant au cours du temps par modification d'un courant de pompage (I) d'une diode de laser du laser à diodes (4), l'intensité de la lumière sortant de l'échantillon (2) étant mesurée, et la concentration du gaz dans l'échantillon (2) étant calculée selon la valeur en fonction du temps du courant de pompage (I) et de la valeur en fonction du temps de l'intensité mesurée, en plus d'un spectre d'absorption une température sur la paroi externe du récipient d'échantillon (13) étant mesurée et prise en compte dans le calcul de la concentration du gaz, et le courant de pompage (I) présentant une première période (9) avec un courant de base (10) essentiellement constant et différent de zéro et une deuxième période (11) avec une rampe de courant (12) partant du courant de base (10) croissant en continu, le gaz prédéfini étant l'azote, un élargissement des raies dans le spectre d'absorption déterminé à partir de la distribution d'intensité selon le courant de pompage étant déterminé et à partir de cet élargissement de raies la pression partielle de vapeur d'eau dans l'échantillon étant renseignée, le calcul de la concentration en azote comprenant un paramètre calibré dont la valeur a été préalablement déterminée par calibration avec au moins un échantillon de référence avec une concentration connue en azote.

2. Procédé selon la revendication 1, **caractérisé en ce que** le courant de base (10) est supérieur ou égal à la valeur de seuil du laser de la diode de laser.

3. Procédé selon la revendication 1 ou 2, **caractérisé en ce que** le courant de pompage (I) présente après la deuxième période (11) une troisième période (29), le courant de pompage (I) diminuant dans la troisième période (29) au moins en deux étapes (31) jusqu'au courant de base (10).

4. Procédé selon l'une des revendications 1 à 3, **caractérisé en ce que** la lumière émise par le laser à diodes (4) est diffusée avant l'entrée dans l'échantillon (2) et/ou après la sortie de l'échantillon (2).

5. Procédé selon la revendication 4, **caractérisé en ce que** la lumière émise par le laser à diodes (4) est concentrée avant ou juste après qu'elle est diffusée.

6. Procédé selon l'une des revendications 1 à 5, **caractérisé en ce que** la lumière laser émise par le laser à diodes (4) présente une longueur d'onde de 1 300 à 2 000 nm, de préférence de 1 750 à 1 950 nm, en particulier de 1 810 à 1 850 nm.

7. Appareil (1) pour la détermination de la concentration en azote dans un échantillon (2) dans une bouteille fermée, l'appareil (1) comprenant un laser à diodes (4) avec une diode de laser pour le rayonnement de lumière essentiellement monochromatique dans l'échantillon (2), un détecteur de lumière (6) pour la mesure de l'intensité de la lumière sortant de l'échantillon (2), une unité de commande (7) pour la commande du courant de pompage (I) de la diode de laser, et une unité d'évaluation (8), l'unité de commande (7) étant configurée pour faire varier au cours du temps le courant de pompage (I) de la diode de laser et l'unité d'évaluation (8) étant raccordée au détecteur de lumière (6) et à l'unité de commande (7) et configurée pour le calcul de la concentration en azote selon le courant de pompage (I) prédéterminé par l'unité de commande (7) et l'intensité mesurée par le détecteur de lumière (6), l'appareil (1) comprenant un dispositif de mesure de température (20), lequel est configuré pour la mesure de la température sur une paroi externe de la bouteille, et l'unité de commande (7) étant configurée pour attribuer au courant de pompage (I) pendant une première période (9) un courant de base (10) essentiellement constant et différent de zéro et pendant une deuxième période (11) une rampe de courant (12) partant du courant de base (10) croissant en continu, l'unité d'évaluation (8) étant configurée pour déterminer un élargissement de raies dans un spectre d'absorption déterminé à partir de l'intensité mesurée selon le courant de pompage et à partir de cet élargissement de raies renseigner sur la pression partielle de vapeur d'eau dans l'échantillon, le calcul de la concentration en azote comprenant un paramètre calibré dont la valeur a été préalablement déterminée par calibration avec au moins un échantillon de référence avec une concentration connue en azote.

8. Appareil (1) selon la revendication 7, **caractérisé en ce que** l'unité de commande (7) est configurée pour attribuer au courant de base (10) un courant de pompage (I) supérieur ou égal à la valeur de seuil du laser de la diode de laser.

9. Appareil (1) selon la revendication 7 ou 8, **caractérisé en ce que** l'unité de commande (7) est configurée pour attribuer pendant une troisième période (29) après la deuxième période (11) un courant de pompage (I) diminuant au moins en deux étapes (31) jusqu'au courant de base (10).

10. Appareil (1) selon l'une des revendications 7 à 9, **caractérisé en ce que** l'appareil (1) comprend au moins un élément de diffusion (23, 24), lequel est configuré pour la diffusion de la lumière rayonnant à partir de la diode de laser avant l'entrée dans l'échantillon (2) et/ou après la sortie de l'échantillon (2).

11. Appareil (1) selon la revendication 10, **caractérisé en ce que** l'appareil (1) comprend au moins une lentille convergente (25, 26), laquelle est configurée pour concentrer la lumière rayonnant à partir de la diode de laser avant la diffusion sur ou dans l'élément de diffusion (23, 24) ou après celui-ci.

12. Appareil (1) selon l'une des revendications 7 à 11, **caractérisé en ce que** le laser à diodes (4) est configuré pour l'émission de lumière laser d'une longueur d'onde de 1 300 à 2 000 nm, de préférence de 1 750 à 1 950 nm, en particulier de 1 810 à 1 850 nm.
